# EUROPEAN PATENT APPLICATION

(11) **EP 4 242 290 A1**
(43) Date of publication of application: **13.09.2023**
(21) Application number: 21889000.2
(22) Date of filing: 14.10.2021
(51) Int. Cl.: C12M 1/04, C12M 1/02, C12M 1/36, C12M 1/38, C12N 1/14, C12N 1/16, C12N 1/20

(54) **CULTURE APPARATUS AND CULTURE METHOD**

(30) Priority: 06.11.2020 JP 2020185897
(71) Applicant: AISIN CORPORATION, Aichi 448-8650 (JP)
(72) Inventor: TABATA Yuki, Kariya-shi, Aichi 448-8650 (JP); OTSUBO Isao, Kariya-shi, Aichi 448-8650 (JP); HIRANO Akiyoshi, Kariya-shi, Aichi 448-8650 (JP)
(74) Representative: Cabinet Beau de Loménie
(86) International application number: PCT/JP2021/038134
(87) International publication number: WO 2022/097447

(57) **Abstract**

A culture apparatus 10 that cultures a subject to be cultured by supplying moisture to the subject to be cultured includes a fine water generating cartridge 30 that is disposed in an air passage 21 by which moisture can be supplied into a culture space 51 and that goes into a moisture absorption state in which moisture in air is adsorbed on conductive polymer films 34b by reducing the temperature of a base material 34a having the conductive polymer films 34b, and goes into a moisture release state in which fine water with a particle size of 50 nanometers or less is released from the moisture adsorbed on the conductive polymer films 34b by increasing the temperature of the base material 34a; and a control part 60 that performs moisture absorption control in which the fine water generating cartridge 30 is brought into the moisture absorption state, and moisture release control in which the fine water released by bringing the fine water generating cartridge 30 into the moisture release state is supplied into the culture space 51, by which the subject to be cultured is irradiated with the fine water.

## Description

### TECHNICAL FIELD

The present disclosure relates to a culture apparatus and a culture method.

### BACKGROUND ART

Conventionally, microorganisms such as molds, yeasts, and bacteria are widely used in various fields such as food and medicine. For example, Patent Literature 1 describes a system for culturing shiitake fungi that includes an apparatus that humidifies intake air and blows the air into a culture room, and grows (cultures) shiitake fungi while maintaining humidity in the culture room at a predetermined value or more. In addition, Patent Literature 2 describes that cheese is inoculated with a beni-koji fungus and the beni-koji fungus is cultured at a predetermined humidity and a predetermined temperature, by which a surface of the cheese is covered with the beni-koji fungus.

### CITATIONS LIST

### PATENT LITERATURE

Patent Literature 1: JP 2005-137273 A
Patent Literature 2: JP 2871882 B2

### SUMMARY OF INVENTION

### TECHNICAL PROBLEMS

As described above, to culture a subject to be cultured, it is important to provide a suitable environment, e.g., humidity is appropriately controlled. Note, however, that it is conceivable that depending on the particle size of water droplets contained in air, absorption of moisture by the subject to be cultured does not proceed, making it hard to promote culture. Hence, besides simply controlling humidity, there is still room for improvement to promote culture.

The present disclosure promotes culture by more appropriately supplying moisture to a subject to be cultured.

### SOLUTIONS TO PROBLEMS

The present disclosure adopts the following means to achieve the above-described promotion of culture.

A culture apparatus of the present disclosure is
a culture apparatus that cultures a subject to be cultured disposed in a culture space by supplying moisture to the subject to be cultured, and includes:
a fine water generating part that is disposed in a passage by which moisture can be supplied into the culture space and that goes into a moisture absorption state in which moisture in air is adsorbed on a conductive polymer film by reducing a temperature of a base material having the conductive polymer film, and goes into a moisture release state in which fine water with a particle size of 50 nanometers or less is released from the moisture adsorbed on the conductive polymer film by increasing the temperature of the base material; and
a control part that performs moisture absorption control in which the fine water generating part is brought into the moisture absorption state, and moisture release control in which the fine water released by bringing the fine water generating part into the moisture release state is supplied into the culture space, by which the subject to be cultured is irradiated with the fine water.

The culture apparatus of the present disclosure performs moisture absorption control in which the fine water generating part is brought into the moisture absorption state, and moisture release control in which fine water released by bringing the fine water generating part into the moisture release state is supplied into the culture space, by which the subject to be cultured is irradiated with the fine water. By this, the subject to be cultured can be cultured by being irradiated with fine water with a particle size of 50 nanometers or less. Fine water with such a particle size is considered to be a size in which the subject to be cultured easily absorbs moisture, and thus, by more appropriately supplying moisture to the subject to be cultured, culture can be promoted.

A culture method of the present disclosure is
a culture method in which a subject to be cultured disposed in a culture space is cultured by supplying moisture to the subject to be cultured, and includes:
a moisture absorbing step of allowing moisture in air to be adsorbed on a conductive polymer film by reducing a temperature of a base material having the conductive polymer film; and
a moisture releasing step of providing a moisture release state in which fine water with a particle size of 50 nanometers or less is released from the moisture adsorbed on the conductive polymer film by increasing the temperature of the base material, and supplying the released fine water into the culture space, by which the subject to be cultured is irradiated with the fine water.

The culture method of the present disclosure performs a moisture absorbing step of allowing moisture in air to be adsorbed on a conductive polymer film by reducing the temperature of a base material having the conductive polymer film; and a moisture releasing step of providing a moisture release state in which fine water with a particle size of 50 nanometers or less is released from the moisture adsorbed on the conductive polymer film by increasing the temperature of the base material, and supplying the released fine water into the culture space, by which the subject to be cultured is irradiated with the fine water. By this, as with the above-described culture apparatus, the subject to be cultured can be cultured by being irradiated with fine water with a particle size of 50 nanometers or less, and thus, by more appropriately supplying moisture to the subject to be cultured, culture can be promoted.

### BRIEF DESCRIPTION OF DRAWINGS

FIG. 1 is a configuration diagram showing a rough configuration of a culture apparatus 10.
FIG. 2A is a configuration diagram showing a rough configuration of a fine water generating cartridge 30, and FIG. 2B is a configuration diagram showing a rough configuration of a fine water generating element 34.
FIG. 3 is a flowchart showing an example of a fine water supplying process.
FIG. 4 is an illustrative diagram showing a state of operation performed upon performing moisture absorption control.
FIG. 5 is an illustrative diagram showing a state of operation performed upon performing moisture release control.
FIG. 6 is an illustrative diagram showing a state of operation performed upon performing humidity adjustment control.
FIG. 7 is a photograph showing a result of culture in an implementation example.
FIG. 8 is a photograph showing a result of culture in comparative example 1.
FIG. 9 is a photograph showing a result of culture in comparative example 2.

### DESCRIPTION OF EMBODIMENTS

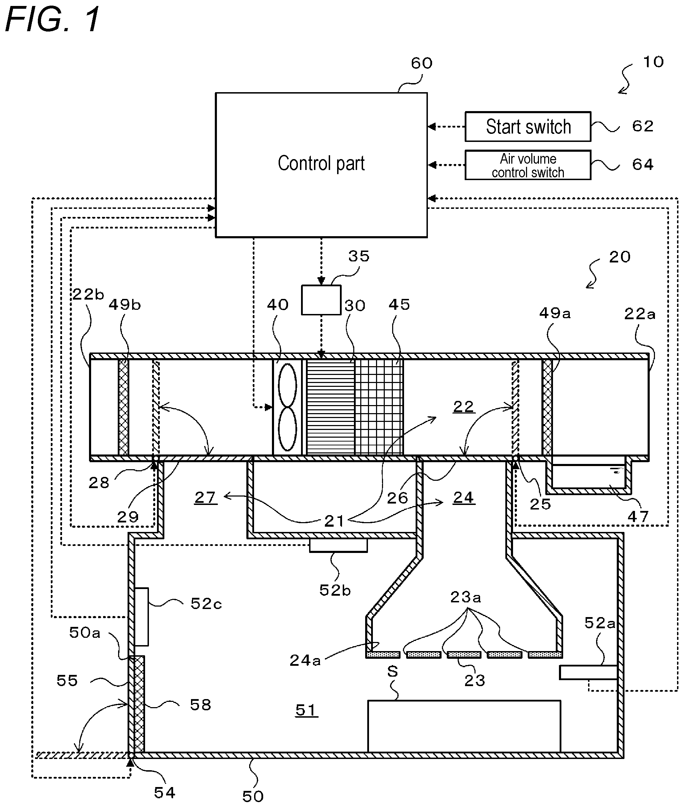

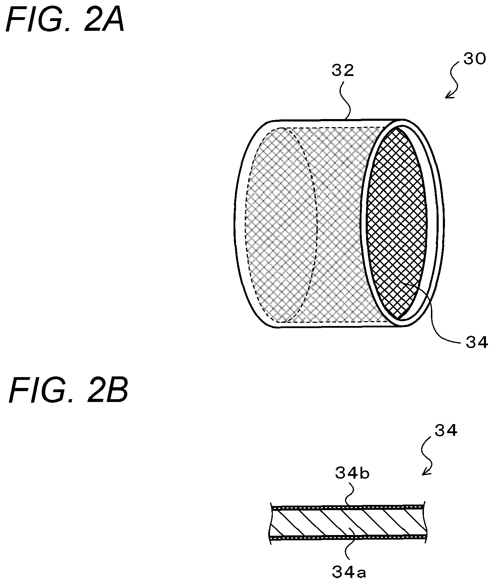

Next, a mode for carrying out the present disclosure will be described. FIG. 1 is a configuration diagram showing a rough configuration of a culture apparatus 10, and FIG. 2 is a configuration diagram showing a rough configuration of a fine water generating cartridge 30. The culture apparatus 10 is configured as an apparatus that promotes culture by supplying fine water, and includes a fine water supply unit 20 that supplies fine water; a culture case 50 in which a container such as a petri dish S that holds a subject to be cultured is disposed in culture space 51; and a control part 60 that controls the entire apparatus. Note that the subject to be cultured includes living cells, pieces of tissue, etc., in addition to microorganisms such as molds, yeasts, and bacteria.

The fine water supply unit 20 includes an air passage 21, the fine water generating cartridge 30, an energization circuit 35, a fan 40, and a temperature control cartridge 45. The air passage 21 includes a main passage 22, a first communicating passage 24, and a second communicating passage 27, and is provided with a first switching part 25, a second switching part 28, a water tank 47, and filters 49a and 49b. The main passage 22 is a cylindrical passage with open ends. In the main passage 22, the water tank 47, the filter 49a, the temperature control cartridge 45, the fine water generating cartridge 30, the fan 40, and the filter 49b are provided in this order from a first opening 22a on one side of the main passage 22 to a second opening 22b on the other side of the main passage 22.

The first communicating passage 24 and the second communicating passage 27 are passages that provide communication between the main passage 22 and the culture case 50 (culture space 51). The first communicating passage 24 is connected to the main passage 22 at a location between the filter 49a on a first opening 22a side and the temperature control cartridge 45, and extends into the culture space 51. The first communicating passage 24 is formed to have a larger inside diameter on an outlet 24a side thereof in the culture space 51 than a side of the first communicating passage 24 connected to the main passage 22. In addition, in the culture space 51, the petri dish S is disposed below the outlet 24a of the first communicating passage 24. The outlet 24a is formed in a hood-like manner so as to cover the petri dish S, and a perforated plate 23 having a plurality of through-holes 23a made therein is attached to the outlet 24a. The second communicating passage 27 is connected to the main passage 22 at a location between the filter 49b on a second opening 22b side and the fan 40, and is connected to the culture case 50.

The first switching part 25 has a switching plate 26 that is operated by drive of a motor which is not shown. When the switching plate 26 is located in a normal position (initial position) that forms a part of an interior wall of the main passage 22, the first switching part 25 closes (shuts) the first communicating passage 24, and opens the first opening 22a side to allow air distribution through the first opening 22a (see a solid line of FIG. 1). In addition, when the switching plate 26 is operated, by drive of the motor, to an operating position that is rotated by 90 degrees from the normal position, the first switching part 25 opens the first communicating passage 24, and closes the first opening 22a side to block air distribution through the first opening 22a (see a dotted line of FIG. 1).

The second switching part 28 has a switching plate 29 that is operated by drive of a motor which is not shown. When the switching plate 29 is located in a normal position (initial position) that forms a part of the interior wall of the main passage 22, the second switching part 28 closes (shuts) the second communicating passage 27, and opens the second opening 22b side to allow air distribution through the second opening 22b (see a solid line of FIG. 1). In addition, when the switching plate 29 is operated, by drive of the motor, to an operating position that is rotated by 90 degrees from the normal position, the second switching part 28 opens the second communicating passage 27, and closes the second opening 22b side to block air distribution through the second opening 22b (see a dotted line of FIG. 1).

As shown in FIG. 2A, the fine water generating cartridge 30 includes a cylindrical case 32 whose outside diameter allows its disposition in the main passage 22; and a fine water generating element 34 provided in the case 32. As shown in FIG. 2B, the fine water generating element 34 includes a base material 34a and conductive polymer films 34b formed on surfaces of the base material 34a.

The base material 34a is made of a material having conductivity such as a metallic material, e.g., a stainless steel-based metal or a copper-based metal, a carbon material, or a conductive ceramic material. In the present embodiment, a metal foil made of stainless steel having aluminum added thereto is used. Note that the fine water generating element 34 is formed in a corrugated plate-like manner, in a honeycomb pattern, in a spiral pattern, etc., so as to allow air distribution and to obtain the largest possible surface area of the base material 34a (conductive polymer films 34b). The energization circuit 35 including a power supply and a switch is connected to the base material 34a. When the switch is turned on, the energization circuit 35 goes into an energization state in which electric current passes through the base material 34a, and when the switch is turned off, the energization circuit 35 goes into a non-energization state in which the passage of electric current through the base material 34a is interrupted. The energization circuit 35 (switch) is turned on and off by the control part 60.

The conductive polymer films 34b are made of a polymer compound having conductivity such as a thiophene-based conductive polymer. In the present embodiment, the conductive polymer films 34b are made of poly(3,4-ethylenedioxythiophene)/poly(styrene sulfonate) (PEDOT/PSS) among thiophene-based conductive polymers. PEDOT/PSS has a core-shell structure having PEDOT as a core and a sulfonate group as a shell, the sulfonate group being an acid functional group capable of hydrogen bonding. In addition, in the conductive polymer films 34b, a layered structure in which PEDOT/PSS shells are aligned is formed, and a nano-channel which is a channel in nanometer size, e.g., 2 nanometers, is formed between the shells. In the nano-channel, many sulfonate groups are present, and thus, when a surface of the conductive polymer film 34b has high moisture content and the inside of the conductive polymer film 34b has low moisture content, due to a difference in concentration between the surface and the inside, the moisture present on the surface of the conductive polymer film 34b moves to the inside along the sulfonate groups in the nano-channel. By this, the conductive polymer film 34b adsorbs moisture. In addition, when, with moisture being adsorbed inside, the surface has low moisture content and the inside has high moisture content, due to a difference in concentration between the surface and the inside, the moisture moves to the surface along the sulfonate groups in the nano-channel. By this, the moisture is released as fine water from the conductive polymer film 34b. In addition, in a state in which the temperature of the conductive polymer film 34b is increased, prompt release of moisture (fine water) is promoted compared to a case in which moisture moves only by a difference in concentration, and in a state in which the temperature of the conductive polymer film 34b is reduced, prompt adsorption of moisture is promoted compared to a case in which moisture moves only by a difference in concentration. As such, the fine water generating cartridge 30 (fine water generating element 34) changes, by a reduction in temperature, to a moisture absorption state in which moisture in air is adsorbed on the conductive polymer films 34b, and changes, by an increase in temperature, to a moisture release state in which the adsorbed moisture is released from the conductive polymer films 34b. Note that the thickness of the conductive polymer films 34b can be determined as appropriate, based on a required amount of fine water adsorbed (a required amount of fine water released). For example, when the conductive polymer films 34b are formed to a thickness of 1 to 30 micrometers, etc., sufficient moisture to release fine water can be adsorbed for a period of about a few seconds to a few tens of seconds.

In addition, the fine water generating cartridge 30 releases uncharged fine water whose water particle size is 50 nanometers or less, e.g., 2 nanometers or less, from the conductive polymer films 34b of the fine water generating element 34. A reason that such particle size is obtained is considered to result from a phenomenon in which since the size of the nano-channel is 2 nanometers or less, due to an improvement in mobility and an increase in pressure of water in the nano-channel by an increase in the temperature of the conductive polymer films, moisture pops out of the nano-channel. In addition, even if water particles are agglomerated after popping out, the particle size thereof is distributed in a range of 50 nanometers or less. A detailed description of such generation of fine water by the fine water generating cartridge 30 (conductive polymer films 34b) is described in the specification of Japanese Patent Application No. 2018-172166 filed by the applicant of the present application, etc., and thus, further detailed description is omitted.

The fan 40 blows air sucked into the main passage 22 from the first opening 22a through the filter 49a, toward the second opening 22b side by rotational drive in a first rotating direction. In addition, the fan 40 blows air sucked into the main passage 22 from the second opening 22b through the filter 49b, toward the first opening 22a side by rotational drive in a second rotating direction which is opposite to the first rotating direction. The fan 40 is rotationally driven by a motor which is not shown, and is pulse width modulation (PWM) controlled by the control part 60. Note that the fan 40 may be a propeller fan or may be a sirocco fan, etc.

The temperature control cartridge 45 is made of a metallic material and formed, for example, in a corrugated plate-like manner, in a honeycomb pattern, or in a spiral pattern so as to have a large heat capacity and high heat exchange efficiency. As described above, the temperature control cartridge 45 is disposed more to the first opening 22a side (the right side of FIG. 1) than the fine water generating cartridge 30. Hence, air having passed through the fine water generating cartridge 30 and having flown into the temperature control cartridge 45 by rotational drive in the second rotating direction of the fan 40 is cooled when passing through the temperature control cartridge 45.

The water tank 47 is formed between the first opening 22a and the filter 49a so as to be able to store water therein, and evaporated moisture is released into the main passage 22. The moisture passes through the filter 49a together with air sucked from the first opening 22a, and flows toward the fine water generating cartridge 30 by rotational drive in the first rotating direction of the fan 40.

The culture case 50 includes humidity sensors 52a to 52c that detect humidity in the culture space 51; a communicating opening 50a that is made in a part of a side wall (e.g., a left wall) of the culture case 50 to provide communication between the inside and outside of the culture space 51; a switching part (open/close part) 54 that switches between opening and closing of the communicating opening 50a; and a filter 58 attached to the communicating opening 50a. The humidity sensor 52a detects humidity near the outlet 24a of the first communicating passage 24, i.e., humidity of air supplied to the petri dish S. The humidity sensor 52b detects humidity in the top of the culture space 51. The humidity sensor 52c detects humidity near the communicating opening 50a.

The switching part 54 has a switching plate 55 that is operated by drive of a motor which is not shown. When the switching plate 55 is located in a normal position (initial position) that forms a part of the side wall of the culture case 50, the switching part 54 closes the communicating opening 50a and interrupts communication between the inside and outside of the culture space 51 (see a solid line of FIG. 1). In addition, when the switching plate 55 is operated, by drive of the motor, to an operating position that is rotated outwardly by 90 degrees from the normal position, the switching part 54 opens the communicating opening 50a to provide communication between the inside and outside of the culture space 51 (see a dotted line of FIG. 1).

The control part 60 is formed as a microprocessor with a CPU being a core, and includes a ROM, a RAM, and input and output ports in addition to the CPU. To the control part 60 there are inputted, through the input ports, an operation signal from a start switch 62, an operation signal from an air volume control switch 64, detection signals (humidity H) from the humidity sensors 52a to 52c, etc. Here, the start switch 62 is a switch for starting operation of the culture apparatus 10. The air volume control switch 64 is a switch for controlling the volume of air blown from the fan 40 to the culture case 50. In addition, from the control part 60 there are outputted, through the output ports, a drive signal to the motor that rotationally drives the fan 40, a drive signal to the switch in the energization circuit 35, drive signals to the motors for the first switching part 25, the second switching part 28, and the switching part 54, etc.

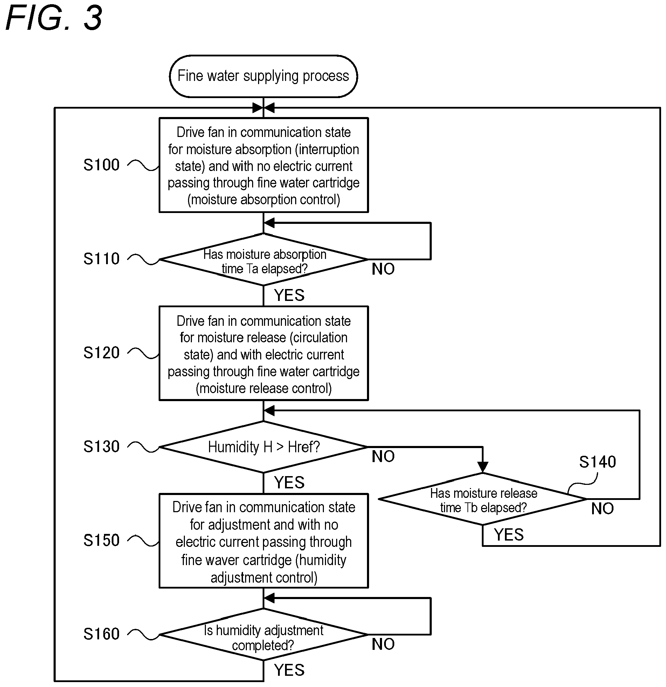

Next, operation of the culture apparatus 10 thus configured will be described. FIG. 3 is a flowchart showing an example of a fine water supplying process. The flowchart is performed by the control part 60 when the start switch 62 is operated, with the power to the culture apparatus 10 being turned on. In the fine water supplying process, the control part 60 first performs moisture absorption control (normal moisture absorption control) in which moisture is adsorbed on the conductive polymer films 34b by driving the fan 40 so as to have a predetermined air volume (first air volume), with the air passage 21 being in a communication state for moisture absorption and with no electric current passing through the fine water generating cartridge 30 (S100), and waits until a predetermined moisture absorption time Ta has elapsed (S110).

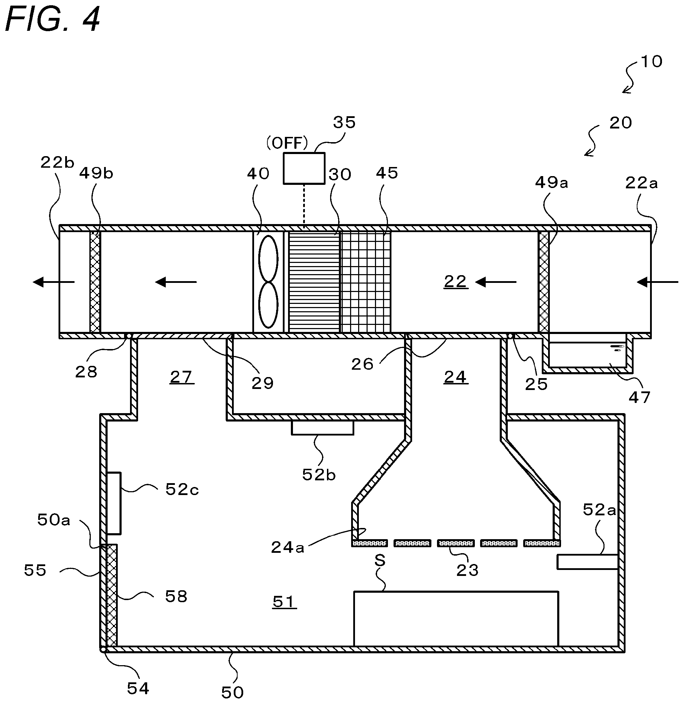

FIG. 4 is an illustrative diagram showing a state of operation performed upon performing moisture absorption control. As shown in the drawing, the first switching part 25 closes the first communicating passage 24 to open the first opening 22a side, and the second switching part 28 closes the second communicating passage 27 to open the second opening 22b side. In this state, the control part 60 rotationally drives the fan 40 in the first rotating direction. Hence, air sucked into the main passage 22 from the first opening 22a is distributed toward the second opening 22b (see arrows of FIG. 4), and thus, moisture evaporated from the water tank 47 also passes through the filter 49a and flows toward the fine water generating cartridge 30. In addition, the control part 60 turns off the energization circuit 35 (switch). Hence, the energization circuit 35 goes into a non-energization state in which passage of electric current through the base material 34a is interrupted, by which the temperature of the conductive polymer films 34b decreases, promoting adsorption of moisture. As such, in the moisture absorption control, adsorption of moisture onto the fine water generating cartridge 30 is promoted in an interruption state in which communication between the air passage 21 and the culture case 50 (culture space 51) is interrupted.

If the control part 60 determines at S110 that the moisture absorption time Ta has elapsed, then the control part 60 performs moisture release control (normal moisture release control) in which fine water released from the conductive polymer films 34b is supplied into the culture space 51 by driving the fan 40 so as to have an air volume (second air volume) smaller than the predetermined air volume, with the air passage 21 being in a communication state for moisture release and with electric current passing through the fine water generating cartridge 30 (S 120). In addition, when the control part 60 performs the moisture release control, while the control part 60 determines (monitors) whether humidity H in the culture space 51 has exceeded a predetermined humidity Href (S 130), the control part 60 waits until a predetermined moisture release time Tb has elapsed (S 140). Note that for the humidity H, any one of detection values of the three humidity sensors 52a to 52c may be used or a value derived from each detection value (e.g., a highest value or an average value) may be used. In the present embodiment, a detection value of the humidity sensor 52a near the outlet 24a of the first communicating passage 24 is used. Note that a target humidity suitable for culture can be determined by the type of a subject to be cultured, etc., and thus, the predetermined humidity Href can be set as appropriate, based on the target humidity. For example, a value higher by about 10 to 15% relative to the target humidity is set as the predetermined humidity Href. Note that monitoring of the humidity H at S130 may be performed during the moisture absorption control.

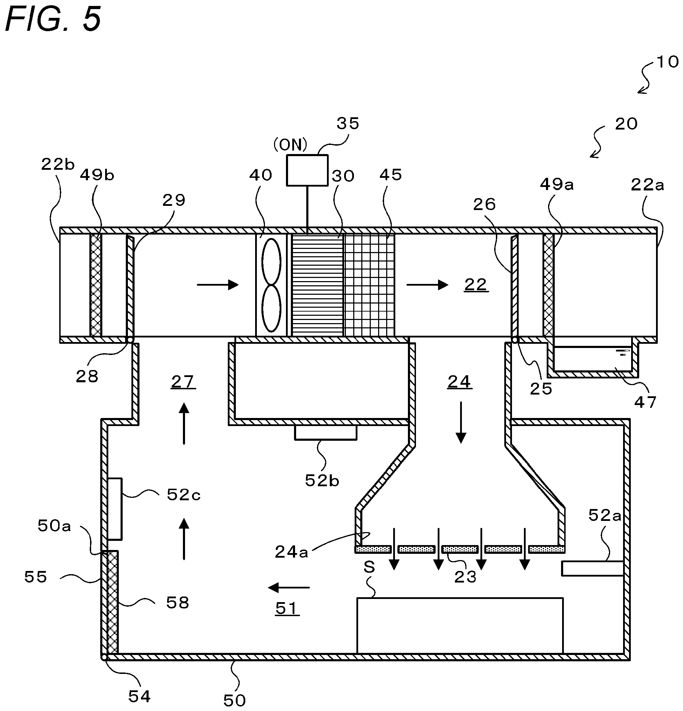

FIG. 5 is an illustrative diagram showing a state of operation performed upon performing moisture release control. As shown in the drawing, the first switching part 25 opens the first communicating passage 24 to close the first opening 22a side, and the second switching part 28 opens the second communicating passage 27 to close the second opening 22b side. Hence, the main passage 22 has no air distribution through the first opening 22a or the second opening 22b, enabling air distribution between the first communicating passage 24 and the second communicating passage 27. In this state, the control part 60 rotationally drives the fan 40 in the second rotating direction. Hence, air blown from the fan 40 passes through the fine water generating cartridge 30 and the temperature control cartridge 45 in turn and flows into the culture space 51 through the first communicating passage 24, and passes through the second communicating passage 27 and returns to the main passage 22. Namely, air flows so as to circulate through the main passage 22, the first communicating passage 24, the culture case 50, and the second communicating passage 27 (see arrows of FIG. 5). In addition, the control part 60 turns on the energization circuit 35 (switch). Hence, the energization circuit 35 goes into an energization state in which electric current passes through the base material 34a, by which the temperature of the conductive polymer films 34b increases, promoting release of fine water. The released fine water is supplied into the culture space 51 by the blown air, and a subject to be cultured in the petri dish S is irradiated with the released fine water. As such, in the moisture release control, the subject to be cultured is irradiated with fine water released from the fine water generating cartridge 30, in a circulation state in which the air passage 21 communicates with the culture space 51 so that air circulates through the air passage 21 and the culture space 51. Note that in the moisture absorption control and the moisture release control, the switching part 54 of the culture case 50 places the switching plate 55 in the normal position to close the communicating opening 50a.

If the control part 60 determines at S140 that the moisture release time Tb has elapsed, then the control part 60 returns to S100 and performs moisture release control. As such, the control part 60 alternately and repeatedly performs moisture absorption control and moisture release control. The moisture absorption time Ta and the moisture release time Tb may be set as appropriate, based on the moisture absorption capability (moisture release capability) of the fine water generating cartridge 30, the size of the culture case 50 (culture space 51), the type of a subject to be cultured, etc. Though not particularly limited, for example, the moisture absorption time Ta is determined to be time that is about twice the moisture release time Tb, and when the moisture release time Tb is 30 seconds or one minute, the moisture absorption time Ta is determined to be one minute, two minutes, etc. In addition, if the control part 60 determines at S130 that the humidity H in the culture space 51 has exceeded the predetermined humidity Href, then the control part 60 performs humidity adjustment control in which outside air is circulated through the culture space 51 by driving the fan 40 with an air volume (second air volume) smaller than the predetermined air volume, with the air passage 21 being in a communication state for adjustment and with no electric current passing through the fine water generating cartridge 30 (S150).

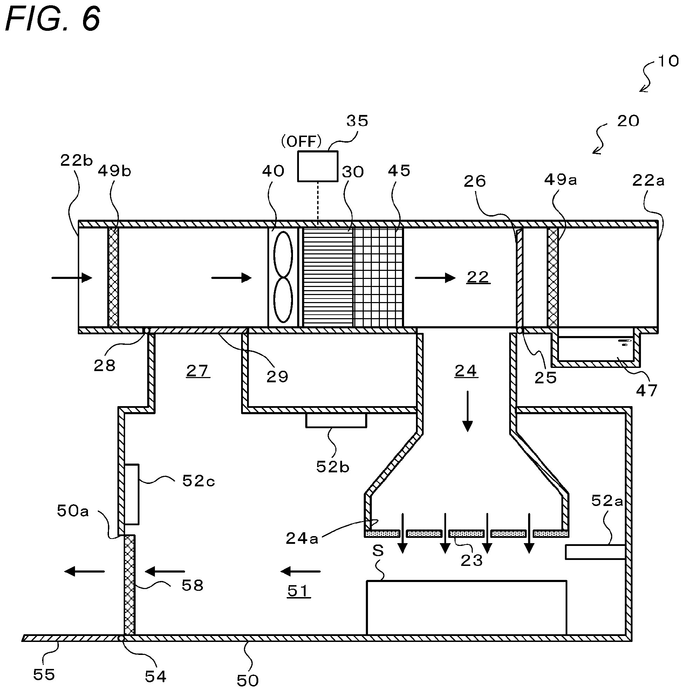

FIG. 6 is an illustrative diagram showing a state of operation performed upon performing humidity adjustment control. As shown in the drawing, as with moisture release control, the first switching part 25 opens the first communicating passage 24 to close the first opening 22a side, and as with moisture absorption control, the second switching part 28 closes the second communicating passage 27 to open the second opening 22b side. In addition, the switching part 54 of the culture case 50 opens the communicating opening 50a to provide communication between the inside and outside of the culture space 51. In this state, the control part 60 rotationally drives the fan 40 in the second rotating direction. Hence, air (outside air) having flown in from the second opening 22b is distributed into the culture space 51 through the first communicating passage 24 and flows out to the outside through the communicating opening 50a (see arrows of FIG. 6). In addition, the control part 60 turns off the energization circuit 35 (switch). Hence, since the temperature of the conductive polymer films 34b decreases, release of fine water is suppressed. As such, in the humidity adjustment control, the humidity in the culture space 51 is adjusted to decrease by providing a communication state (partial communication state) in which the air passage 21 communicates with the culture case 50 (culture space 51) so that air is distributed in one direction, and bringing outside air into the culture case 50 through the air passage 21. Note that the air volume control switch 64 may be allowed to set the air volume of the fan 40 for moisture absorption control, moisture release control, and humidity adjustment control.

When the humidity adjustment control is thus performed, the control part 60 waits until completion of the humidity adjustment (S160), and if the control part 60 determines that the humidity adjustment is completed, then the control part 60 returns to S100 and repeats processes. At S160, it is determined that the humidity adjustment is completed when the humidity H in the culture space 51 has fallen below the predetermined humidity Href or when the humidity H has fallen below humidity for determining completion which is slightly lower than the predetermined humidity Href. Alternatively, it may be determined that the humidity adjustment is completed when time elapsed from the start of humidity adjustment control has reached a predetermined adjustment time.

The culture apparatus 10 described above alternately performs moisture absorption control in which the fine water generating cartridge 30 is brought into a moisture absorption state, and moisture release control in which fine water released by bringing the fine water generating cartridge 30 into a moisture release state is supplied into the culture space 51, and thus, can culture a subject to be cultured by allowing the subject to be cultured to be irradiated with the fine water. Here, since the fine water has a very small particle size of 50 nanometers or less, the fine water is considered to be easily absorbed by the subject to be cultured. Hence, the metabolic efficiency of the subject to be cultured improves, enabling an increase in culture rate, and thus, culture can be promoted. In addition, the culture apparatus 10 can have a compact configuration in which the fine water supply unit 20 and the culture case 50 are integrally formed.

In addition, when moisture absorption control is performed, the first communicating passage 24 and the second communicating passage 27 are closed and the temperature of the fine water generating cartridge 30 is reduced by air passing through the main passage 22, by which moisture absorption can be promoted. In addition, by closing each communicating passage, an air volume suitable for moisture absorption can be obtained without affecting the subject to be cultured. In addition, moisture absorption can be further promoted by using moisture evaporated from the water tank 47. In addition, when moisture release control is performed, since air flows in a circulating manner, fine water is prevented from flowing out to the outside, by which the subject to be cultured can be sufficiently irradiated with the fine water.

In addition, when the humidity H in the culture space 51 has exceeded the predetermined humidity Href, humidity adjustment control is performed, and thus, the humidity H can be easily adjusted to humidity suitable for culture.

In addition, the first communicating passage 24 is formed in a hood-like manner to have a large inside diameter on the outlet 24a side thereof, and in moisture release control, the air volume is smaller than that for moisture absorption control, and thus, the flow velocity of air moving toward the petri dish S is reduced, by which wind pressure applied to the subject to be cultured can be suppressed. In addition, the perforated plate 23 having the plurality of through-holes 23a made therein is attached to the outlet 24a, and thus, air having passed through the first communicating passage 24 can move toward the petri dish S while being diffused through the plurality of through-holes 23a, and can uniformly flow over the entire petri dish S. From these facts, the entire petri dish S can be evenly irradiated with fine water.

In addition, air blown from the fan 40 in moisture release control passes through the temperature control cartridge 45, and thus, after the temperature of the air is increased in the fine water generating cartridge 30, the air is cooled to a temperature close to room temperature by the temperature control cartridge 45. Hence, a temperature increase resulting from flowing of high-temperature air into the culture space 51 can be suppressed, and thus, a temperature suitable for culture can be easily maintained.

In the embodiment, the first communicating passage 24 is formed to have a large inside diameter on the outlet 24a side thereof, and in moisture release control, the air volume is smaller than that for moisture absorption control, but the configuration is not limited thereto. For example, the first communicating passage 24 may be formed to have a large inside diameter on the outlet 24a side thereof, and the air volume may be the same for moisture release control and moisture absorption control, or the first communicating passage 24 may be formed to have a constant inside diameter all the way to the outlet 24a side thereof, and in moisture release control, the air volume may be smaller than that for moisture absorption control. Alternatively, when there is no need to consider the speed of wind that hits the subject to be cultured, e.g., there is a sufficient distance between the outlet 24a and the petri dish S, the first communicating passage 24 may be formed to have a constant inside diameter all the way to the outlet 24a side thereof, and the air volume may be the same for moisture release control and moisture absorption control. In addition, although the perforated plate 23 is attached to the outlet 24a, a filter such as a nonwoven fabric, etc., may be attached instead of a perforated plate. Alternatively, the outlet 24a may be simply open without a perforated plate, a filter, etc., attached thereto.

In the embodiment, when the humidity H in the culture case 50 has exceeded the predetermined humidity Href, humidity adjustment control is performed, but the configuration is not limited thereto, and humidity adjustment control may not be performed. In such a case, the culture case 50 may not have the communicating opening 50a made therein and may not include the switching part 54. In addition, a case in which the humidity H exceeds the predetermined humidity Href may be handled by stopping moisture release control. Note that when the humidity H easily increases, e.g., the humidity H frequently exceeds the predetermined humidity Href, an adjustment, e.g., reducing the moisture release time Tb, may be made. Note that although inclusion of the three humidity sensors 52a to 52c in the culture case 50 is exemplified, one or more humidity sensors may be included.

In the embodiment, the water tank 47 is provided in the main passage 22, but the configuration is not limited thereto, and the water tank 47 may not be provided. Even by this configuration, the fine water generating cartridge 30 can absorb moisture in air and release fine water. In addition, the main passage 22 and the culture case 50 communicate with each other by the first communicating passage 24 and the second communicating passage 27, but the configuration is not limited thereto. For example, the main passage 22 may be configured to directly communicate with the culture case 50 through a switching part. In addition, in the embodiment, the temperature control cartridge 45 is provided in the main passage 22, but the configuration is not limited thereto, and the temperature control cartridge 45 may be provided in the first communicating passage 24. Alternatively, the temperature control cartridge 45 may not be provided. In addition, the culture apparatus 10 is not limited to one including all components of the culture apparatus 10 of the present embodiment, and the culture apparatus 10 may have any configuration as long as the culture apparatus 10 includes the fine water generating cartridge 30 and the control part 60 that performs moisture absorption control in which the fine water generating cartridge 30 is brought into a moisture absorption state, and moisture release control in which the fine water generating cartridge 30 is brought into a moisture release state. By mounting a culture apparatus configured to include the fine water generating cartridge 30 and the control part 60 on a commercially available culture case, culture apparatus, etc., it may function as the culture apparatus 10 of the present embodiment, i.e., an apparatus that cultures a subject to be cultured by supplying fine water with a particle size of 50 nanometers or less to the subject to be cultured.

Although in the embodiment, during a culture period, fine water is continuously supplied (supplied at all times) by alternately performing moisture absorption control and moisture release control, the configuration is not limited thereto. For example, a user may be allowed to set, through a setting operating panel, etc., operating time and non-operating time during which moisture absorption control and moisture release control are not performed, and fine water may be intermittently supplied.

### [Summary of the present embodiment]

A culture apparatus (10) according to the embodiment of the present disclosure is a culture apparatus that cultures a subject to be cultured disposed in a culture space (51) by supplying moisture to the subject to be cultured, and includes a fine water generating part (30) that is disposed in a passage (21) by which moisture can be supplied into the culture space (51) and that goes into a moisture absorption state in which moisture in air is adsorbed on conductive polymer films (34b) by reducing the temperature of a base material (34a) having the conductive polymer films (34b), and goes into a moisture release state in which fine water with a particle size of 50 nanometers or less is released from the moisture adsorbed on the conductive polymer films (34b) by increasing the temperature of the base material (34a); and a control part (60) that performs moisture absorption control in which the fine water generating part (30) is brought into the moisture absorption state, and moisture release control in which the fine water released by bringing the fine water generating part (30) into the moisture release state is supplied into the culture space (51), by which the subject to be cultured is irradiated with the fine water.

By this, the subject to be cultured can be cultured by being irradiated with fine water with a particle size of 50 nanometers or less. Fine water with such a particle size is considered to be a size in which the subject to be cultured easily absorbs moisture, and thus, by more appropriately supplying moisture to the subject to be cultured, culture can be promoted.

In addition, the culture apparatus (10) may include an energizing part (35) that can pass electric current through the fine water generating part (30); and an air blowing part (40) disposed in the passage, and when the control part (60) performs the moisture release control, the control part (60) may control the energizing part (35) and the air blowing part (40) such that the fine water is supplied into the culture space (51) by blowing air by bringing the fine water generating part (30) into the moisture release state by increasing the temperature of the base material (34a) by passage of electric current, and when the control part (60) performs the moisture absorption control, the control part (60) may control the energizing part (35) to bring the fine water generating part (30) into the moisture absorption state by reducing the temperature of the base material (34a) by stopping passage of electric current.

By this, the state of the fine water generating part (30) can be easily switched by whether or not to pass electric current therethrough, and fine water can be sufficiently supplied into the culture space (51) by blowing air, and thus, culture of the subject to be cultured can be further promoted.

In addition, the passage (21) may be provided with a main passage (22) in which the fine water generating part (30) and the air blowing part (40) are disposed; communicating passages (24 and 27) that provide communication between the main passage (22) and the culture space (51); switching parts (25 and 28) that can switch between a circulation state in which the main passage (22) communicates with the culture space (51) through the communicating passages (24 and 27) so that air circulates through the main passage (22) and the culture space (51), and an interruption state in which the communication through the communicating passages (24 and 27) is interrupted; and a water tank part (47) that stores water on a one-end side of the main passage (22), and when the control part (60) performs the moisture absorption control, the control part (60) may control the air blowing part (40) such that air flows into the main passage (22) from the water tank part (47) side and flows through the fine water generating part (30) by bringing the switching parts (25 and 28) into the interruption state, and when the control part (60) performs the moisture release control, the control part (60) may control the air blowing part (40) such that air containing the fine water circulates by bringing the switching parts (25 and 28) into the circulation state.

By this, when moisture absorption control is performed, by using moisture evaporated from the water tank part, the moisture can be sufficiently adsorbed on the conductive polymer films. In addition, since the moisture absorption control is performed in an interruption state, the air blowing part can obtain a suitable air volume for moisture absorption. In addition, since moisture release control is performed in a circulation state, release of fine water released from the fine water generating part to the outside is suppressed, and the subject to be cultured can be irradiated with the fine water, and thus, culture can be further promoted.

In addition, the culture space (51) may be provided with an open/close part (54) that opens and closes a communicating opening that provides communication between the inside and outside of the culture space (51); and humidity sensors (52a to 52c) that detect humidity inside the culture space (51), the switching parts (25 and 28) may be able to switch to a communication state in which one of the communicating passages (24 and 27) is allowed for communication so that air is distributed in one direction between the main passage (22) and the culture space (51), and when the control part (60) performs the moisture absorption control and the moisture release control, the control part (60) may allow the open/close part (54) to close the communicating opening, and when humidity detected by the humidity sensors (52a to 52c) has exceeded a predetermined humidity, the control part (60) may perform humidity adjustment control in which the air blowing part (40) is controlled such that air having flown into the main passage (22) is distributed into the culture space (51) through the communicating passages (24 and 27) and flows out through the communicating opening, by bringing the switching parts (25 and 28) into the communication state and allowing the open/close part (54) to open the communicating opening.

By this, even when the humidity in the culture space exceeds humidity suitable for culture, a humidity adjustment can be easily made, and thus, inhibition of promotion of culture can be suppressed.

In addition, in the moisture absorption control, the control part (60) may control the air blowing part (40) to have a predetermined air volume, and in the moisture release control, the control part (60) may control the air blowing part (40) to have an air volume smaller than the predetermined air volume.

By this, wind pressure applied to the subject to be cultured is suppressed, enabling the subject to be cultured to be appropriately irradiated with fine water.

In addition, the culture apparatus (10) may include a heat exchanging part (45) that is disposed more to a downstream side than the fine water generating part (30) in flow of air in the passage (21) occurring when moisture is supplied into the culture space (51), and that performs heat exchange with air passing through the passage (21).

By this, even if the temperature of the base material is increased to release fine water, flowing of high-temperature air into a casing can be suppressed. Hence, not only humidity but also temperature suitable for culture can be maintained.

In addition, when the control part performs the humidity adjustment control, the control part may bring the fine water generating part (30) into the moisture absorption state by reducing the temperature of the base material (34a) by stopping passage of electric current.

In addition, in the moisture absorption control, the control part may control the air blowing part (40) to have a predetermined air volume, and in the humidity adjustment control, the control part may control the air blowing part (40) to have an air volume smaller than the predetermined air volume.

In addition, the passage (21) may be formed to have a large inside diameter on an outlet side thereof from which air is released into the culture space (51).

In addition, the volume of air moving toward the subject to be cultured from the passage (21) may be small compared to that used when the moisture absorption control is performed.

A culture method according to the embodiment of the present disclosure is a culture method in which a subject to be cultured disposed in a culture space (51) is cultured by supplying moisture to the subject to be cultured, and performs a moisture absorbing step of allowing moisture in air to be adsorbed on conductive polymer films (34b) by reducing the temperature of a base material (34a) having the conductive polymer films (34b); and a moisture releasing step of providing a moisture release state in which fine water with a particle size of 50 nanometers or less is released from the moisture adsorbed on the conductive polymer films (34b) by increasing the temperature of the base material (34a), and supplying the released fine water into the culture space (51), by which the subject to be cultured is irradiated with the fine water.

By this, the subject to be cultured can be cultured by being irradiated with fine water with a particle size of 50 nanometers or less, and thus, by more appropriately supplying moisture to the subject to be cultured, culture can be promoted.

Although the mode for carrying out the present disclosure has been described above, the present disclosure is not limited to such an embodiment at all and can, of course, be implemented in various modes without departing from the spirit and scope of the present disclosure.

### IMPLEMENTATION EXAMPLE

The following describes, as an implementation example, an example in which culture is performed using the culture apparatus 10 of the present disclosure. Note that the present disclosure is not limited to the following implementation example.

### (Culture)

A petri dish was used as a culture container, and 20 µL of a suspension of black-koji mold (NBRC4066) was dropped onto potato dextrose agar with chloramphenicol. In the implementation example, the petri dish with an open lid was disposed in the culture case 50 of the culture apparatus 10, and it was irradiated with fine water by performing a fine water supplying process for 24 hours. Note that moisture absorption control and moisture release control were alternately and repeatedly performed, with the above-described moisture absorption time Ta being two minutes and the above-described moisture release time Tb being one minute.

In comparative examples 1 and 2, as in the implementation example, a petri dish was used, and 20 µL of a suspension of black-koji mold (NBRC4066) was dropped onto potato dextrose agar with chloramphenicol. In comparative example 1, the petri dish with a closed lid was left for 24 hours. In addition, in comparative example 2, the petri dish with an open lid was left in a high humidity (95% RH) environment for 24 hours. After a lapse of 24 hours, in all of the implementation example and comparative examples 1 and 2, a lid was placed on the petri dish and the petri dish was left in a constant temperature bath at 30°C for five days.

### (Results)

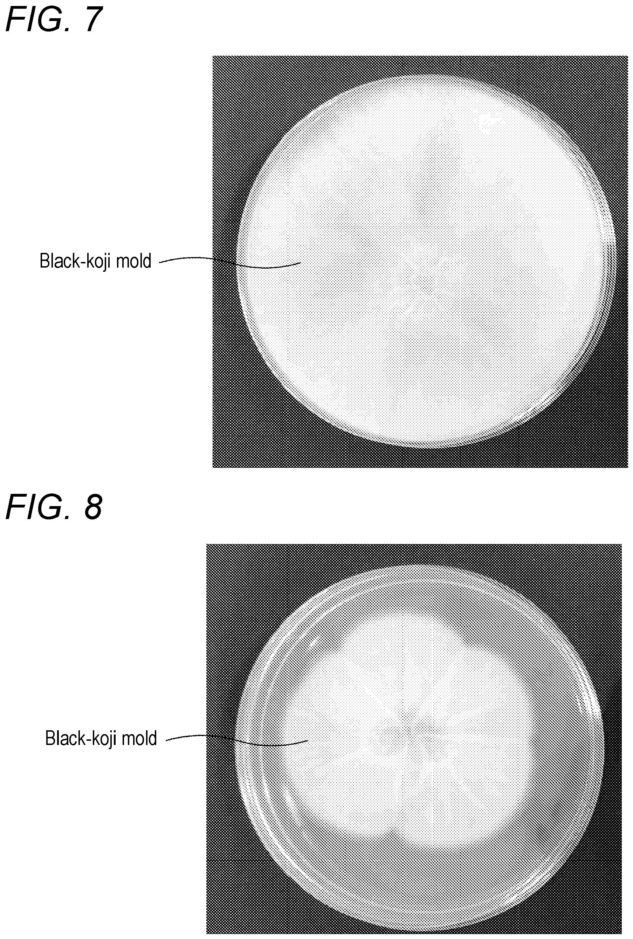

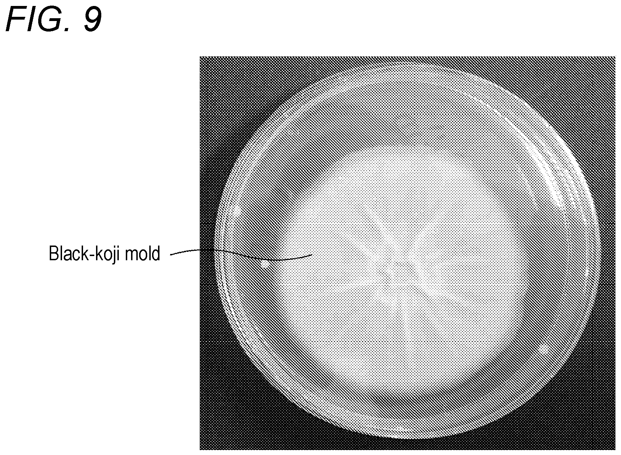

FIG. 7 is a photograph showing a result of culture in the implementation example. FIG. 8 is a photograph showing a result of culture in comparative example 1. FIG. 9 is a photograph showing a result of culture in comparative example 2. These photographs are taken of the petri dishes from above after a lapse of five days from when the petri dishes are left in the constant temperature bath. In comparative example 1 and comparative example 2, as shown in FIGS. 8 and 9, culture was not spread all over the petri dishes but was spread only partially. On the other hand, in the implementation example, as shown in FIG. 7, culture was spread all over the petri dish, exhibiting superiority. As described above, the implementation example shows that culture is promoted by allowing the black-koji mold to be irradiated with fine water.

### INDUSTRIAL APPLICABILITY

The present disclosure can be used in an industry of manufacturing culture apparatuses, etc.

## Claims

1. A culture apparatus that cultures a subject to be cultured disposed in a culture space by supplying moisture to the subject to be cultured, the culture apparatus comprising:
a fine water generating part that is disposed in a passage by which moisture can be supplied into the culture space and that goes into a moisture absorption state in which moisture in air is adsorbed on a conductive polymer film by reducing a temperature of a base material having the conductive polymer film, and goes into a moisture release state in which fine water with a particle size of 50 nanometers or less is released from the moisture adsorbed on the conductive polymer film by increasing the temperature of the base material; and
a control part that performs moisture absorption control in which the fine water generating part is brought into the moisture absorption state, and moisture release control in which the fine water released by bringing the fine water generating part into the moisture release state is supplied into the culture space, by which the subject to be cultured is irradiated with the fine water.

2. The culture apparatus according to claim 1, comprising:
an energizing part that can pass electric current through the fine water generating part; and
an air blowing part disposed in the passage,
wherein
when the control part performs the moisture release control, the control part controls the energizing part and the air blowing part such that the fine water is supplied into the culture space by blowing air by bringing the fine water generating part into the moisture release state by increasing the temperature of the base material by passage of electric current, and when the control part performs the moisture absorption control, the control part controls the energizing part to bring the fine water generating part into the moisture absorption state by reducing the temperature of the base material by stopping passage of electric current.

3. The culture apparatus according to claim 2, wherein
the passage is provided with a main passage in which the fine water generating part and the air blowing part are disposed; a communicating passage that provides communication between the main passage and the culture space; a switching part that can switch between a circulation state in which the main passage communicates with the culture space through the communicating passage so that air circulates through the main passage and the culture space, and an interruption state in which the communication through the communicating passage is interrupted; and a water tank part that stores water on a one-end side of the main passage, and
when the control part performs the moisture absorption control, the control part controls the air blowing part such that air flows into the main passage from the water tank part side and flows through the fine water generating part by bringing the switching part into the interruption state, and when the control part performs the moisture release control, the control part controls the air blowing part such that air containing the fine water circulates by bringing the switching part into the circulation state.

4. The culture apparatus according to claim 3, wherein
the culture space is provided with an open/close part that opens and closes a communicating opening that provides communication between inside and outside of the culture space; and a humidity sensor that detects humidity inside the culture space,
the switching part can switch to a communication state in which a part of the communicating passage is allowed for communication so that air is distributed in one direction between the main passage and the culture space, and
when the control part performs the moisture absorption control and the moisture release control, the control part allows the open/close part to close the communicating opening, and when humidity detected by the humidity sensor has exceeded a predetermined humidity, the control part performs humidity adjustment control in which the air blowing part is controlled such that air having flown into the main passage is distributed into the culture space through the communicating passage and flows out through the communicating opening, by bringing the switching part into the communication state and allowing the open/close part to open the communicating opening.

5. The culture apparatus according to any one of claims 2 to 4, wherein in the moisture absorption control, the control part controls the air blowing part to have a predetermined air volume, and in the moisture release control, the control part controls the air blowing part to have an air volume smaller than the predetermined air volume.

6. The culture apparatus according to any one of claims 1 to 5, comprising a heat exchanging part that is disposed more to a downstream side than the fine water generating part in flow of air in the passage occurring when moisture is supplied into the culture space, and that performs heat exchange with air passing through the passage.

7. The culture apparatus according to claim 4, wherein when the control part performs the humidity adjustment control, the control part brings the fine water generating part into the moisture absorption state by reducing the temperature of the base material by stopping passage of electric current.

8. The culture apparatus according to claim 4, wherein in the moisture absorption control, the control part controls the air blowing part to have a predetermined air volume, and in the humidity adjustment control, the control part controls the air blowing part to have an air volume smaller than the predetermined air volume.

9. The culture apparatus according to claim 5, wherein the passage is formed to have a large inside diameter on an outlet side of the passage from which air is released into the culture space.

10. The culture apparatus according to any one of claims 5, 8, and 9, wherein a volume of air moving toward the subject to be cultured from the passage is small compared to a volume of air used when the moisture absorption control is performed.

11. A culture method in which a subject to be cultured disposed in a culture space is cultured by supplying moisture to the subject to be cultured, the culture method comprising:
a moisture absorbing step of providing a moisture absorption state in which moisture in air is adsorbed on a conductive polymer film by reducing a temperature of a base material having the conductive polymer film; and
a moisture releasing step of providing a moisture release state in which fine water with a particle size of 50 nanometers or less is released from the moisture adsorbed on the conductive polymer film by increasing the temperature of the base material, and supplying the released fine water into the culture space, by which the subject to be cultured is irradiated with the fine water.
